# EUROPEAN PATENT APPLICATION

(11) **EP 0 821 939 A1**
(43) Date of publication of application: **04.02.1998**
(21) Application number: 96830424.6
(22) Date of filing: 31.07.1996
(51) Int. Cl.: A61K 7/42

(54) **Sun screening preparations comprising triazine derivatives**

(71) Applicant: 3V SIGMA S.p.A, I-20121 Milano (IT)
(72) Inventor: Malpede, Alverio, 24121 Bergamo (IT)
(74) Representative: Bianchetti, Giuseppe

(57) **Abstract**

Cosmetic compositions for the protection of human skin from sun radiation, having synergistic activity, are described. Said cosmetic compositions comprise in admixture with conventional vehicles and excipients a combination of at least a compound of formula I 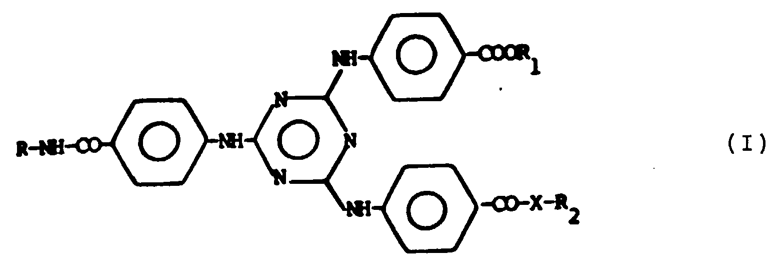 wherein
R is C₁-C₈ straight or branched alkyl, C₅-C₁₂ cycloalkyl, optionally substituted with one or more C₁-C₄ alkyl groups;
X is oxygen or the NH- group;
R₁ has the same meanings of R or is hydrogen, an alkali metal, an ammonium group or a group of formula III 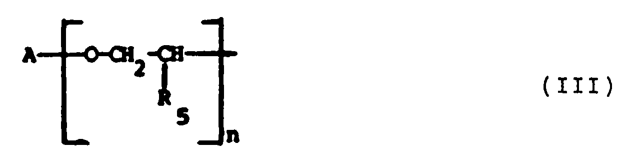 wherein
A is C₁-C₈ straight or branched alkyl, C₅-C₈ cycloalkyl, aryl, optionally substituted with one or more C₁-C₄ alkyls, R₅ is hydrogen or methyl and n can be an integer from 1 to 10;
R₂ has the same meanings of R when X is the NH group or has the same meaning of R₁ when X is oxygen;
   with at least a compound of formula II 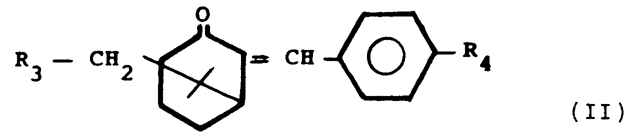 wherein
R₃ is hydrogen, or the SO₃M group, wherein M is an alkali metal or an amine group, R₄ is methyl, a SO₃M group, or a group of formula IV or V

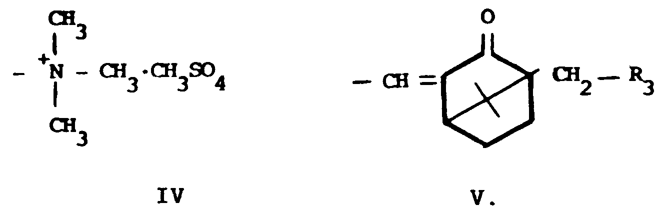

## Description

The present invention relates to cosmetic compositions (hereinafter called sun-protecting formulations) for the protection of skin and/or hair from ultraviolet radiations, containing new synergistic mixtures of sunscreens.

It is well known that sun radiation ranging from 280 to 400 nm is noxious for human skin; in particular that at wavelength ranging from 280 to 320 nm, the so called UV-B radiation, cause erythema and cutaneous burns, whose severity depends on duration of exposure and the kind of the skin.

It has been ascertained that also radiations ranging from 320 to 400 nm, so called UV-A and responsible of skin tanning can provoke noticeable alterations and damages to the skin, especially in cases of sensible skin or in case of continuous exposition to radiations.

A number of sun-protecting formulations were proposed for the photoprotection of skin and a very wide patent literature exists in this regard.

Very often these sun-protecting formulations are in the form of oil-in-water emulsion containing one or more organic lipophilic and/or hydrophilic sunscreens, in variable concentrations, capable of absorbing more or less intensely UV radiations of sun light.

The kind of sunscreens and their usable amount are selected depending on the desired sun protecting factor (SPF). SPF is an index of protection and is expressed as the ratio between the time of irradiation necessary to reach the erythematogenic threshold at the presence of the UV filter and the time necessary to reach the erythematogenic threshold in the absence of the UV filter.

It has now been found that the combination of particular sunscreens and precisely of one or more compounds of formula I, as described hereinafter, and of one or more derivatives of benzylidenecamphor of formula II, as described hereinafter, gives sun-protecting formulations an SPF higher than those obtainable by using, at the same concentrations, the compounds of formula I or those of formula II alone.

It is an object of the present invention a cosmetic composition, for the protection of human skin from sun radiation, comprising in admixture with vehicles and conventional excipients a combination of at least a compound of formula I 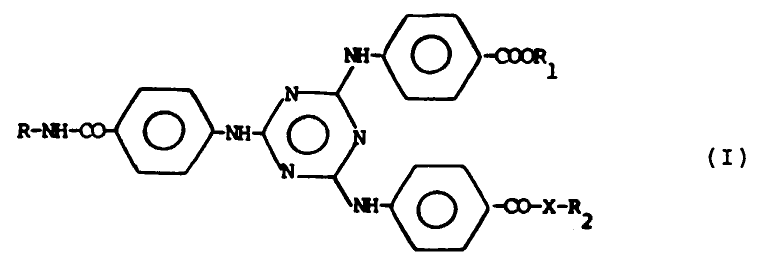 wherein
R is C₁-C₈ straight or branched alkyl, C₅-C₁₂ cycloalkyl optionally substituted with one or more C₁-C₄ alkyl groups;
X is oxygen or the NH- group;
R₁ has the same meanings of R or is hydrogen, an alkali metal, an ammonium group or a group of formula III 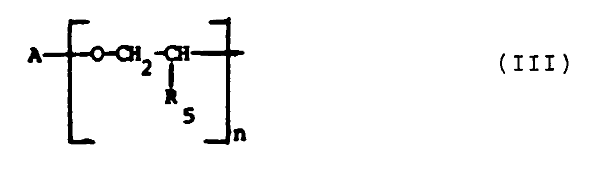 wherein
A is C₁-C₈ straight or branched alkyl, C₅-C₈ cycloalkyl, aryl, which may be optionally substituted with one or more C₁-C₄ alkyls, R₅ is hydrogen or methyl and n can be an integer from 1 to 10;
R₂ has the same meanings of R when X is the group NH or has the same meaning of R₁ when X is oxygen;
   with at least a compound of formula II 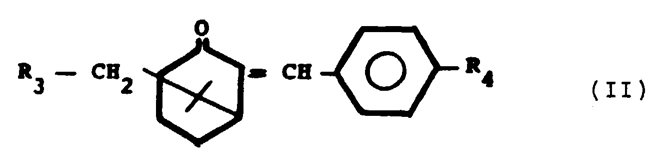 wherein
R₃ is hydrogen, or the SO₃M group, wherein M is an alkali metal or an amine group, R₄ is methyl, a SO₃M group, or a group of formula IV or V

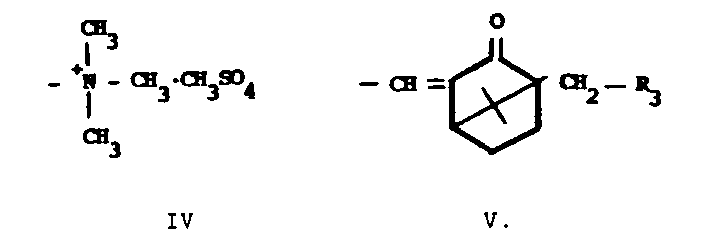

Preferred compounds of formula I are those wherein:
- R= CH₃; R₁=R₂=C₄H₉-CH(C₂H₅)-CH₂-; X=O.
- R=(CH₃)₃C-CH₂-C(CH₃)₂-; R₁=R₂=C₄H₉-CH(C₂H₅)-CH₂-; X=O.
- R=R₂=(CH₃)₂CH; R₁=C₁₄H₂₉; X=NH.
- R=(CH₃)₃C-; R₁=R₂=C₄H₉-CH(C₂H₅)-CH₂-; X=O.

The compounds of formula I are described in US Patent 5346691, whereas the derivatives of benzylidenecamphor of formula II are well known and used since long time, under the trade name of Eusolex 6300 (Merck)^{(R)}, Mesoril SX (L'Oreal).

The present invention relates also to the use of the cosmetic compositions containing combinations of the above sunscreens as photoprotective agents.

According to the present invention, the sun-protecting formulations comprise a cosmetic substrate containing, with respect to the weight of the composition, from 1 to 15% of a derivative of benzylidenecamphor (compound of formula II) and from 0.5 to 10% of a compound of formula I.

The sun-protecting formulations according to the present invention are applied on skin in order to protect it from the noxious action of the UV radiation, thus avoiding erythema or burns formation. Said compositions also serve for the treatment and then also for the protection of hair or of makeup in decorative cosmetics.

According to the present invention, in addition to the compounds of formula I and II, the cosmetic compositions can also contain one or more complementary hydrophilic or lipophilic sunscreens active in UV-B and/or in UV-A.

Examples of these other complementary sunscreens, usually used in cosmetics, are: derivatives of 4-methoxy-cinnamic acid, derivatives of salicylic acid, derivatives of 2-phenyl-benzimidazole, derivatives of 2-cyano-3,3-diphenylacrylic acid, derivatives of benzophenone, 2-ethylhexyl-2-cyano-3,3-diphenylacrylate; 2-ethylhexyl-4-methoxy cinnamate; menthylsalicylate; 2,4,6-trianilino-(p-carbo-2-ethylhexyloxy)-1,3,5-triazine; 2-phenylbenzimidazol-5-sulfonic acid; 4-methoxy-4-ter-butyldibenzoylmethane; 4-isopropyldibenzoylmethane; 2-hydroxy-4-methoxybenzophenone.

The sun-protecting formulations according to the present invention can also contain inorganic pigments usually used in cosmetics, such as for example titanium oxide, zinc oxide, silicon oxide or aluminium oxide.

According to the present invention the cosmetic compositions can be in the form of oil-in-water or water-in-oil emulsions, solutions, lotions, or can be also in the form of gels, lipsticks, aerosol.

The compositions of the present invention are prepared by formulating conventional ingredients, such as for example oils, fats, emulsifiers, hydrating agents, moisturizing agents, emollients, preservatives, surfactants, thickening agents, perfumes, pigments, dyes and other else, such as alcohols, polyols, electrolytes, siliconic derivatives. The more commonly used solvents are triglycerides of caprinic or caprilic acid, castor oil, esters of fatty acids with isopropanol, propylene glycol, glycerin, propylene glycol monomethyl- or monoethyl- or monobutyl ether.

The present invention also comprises the protection of the cosmetic compositions themselves from UV radiation by means of the use of the combinations of the compounds of formula I and II. Said process is characterized in that from 0.5 to 6% of a compound of formula I and from 1 to 10% of a compound of formula II are added to the cosmetic substrate. In this case it is a matter of compositions whose components can undergo degradation or unwanted colouring due to light. Examples in this sense are shampoos, lacquers, hairdress lotions, hair-dye compositions, formulations for make-up, such as nail lacquers, foundation, lipsticks. Preferred cosmetic compositions are those for the protection of human skin from sun radiations.

The following examples further illustrate the invention:

### Example 1

3 sun-protecting formulations having the following composition were prepared:

| | |
|---|---|
| Triglycerides of fatty acid | 20.0 g |
| Cetylstearyl alcohol ethoxylated with 33 moles of ethylene oxide | 2.0 g |
| Cetylstearyl alcohol | 2.0 g |
| Lanoline | 4.0 g |
| Silicone oil | 0.4 g |
| Sunscreen | 5.0 g |
| Abiol (R) (Preservative by 3V-SIGMA) | 0.2 g |
| Synthalen (R) M (crosslinked polyacrylic acid by 3V-SIGMA) | 0.1 g |
| Triethanolamine | 0.15 g |
| Perfume | 0.3 g |
| Distilled water | q.s.to 100.0 |

The fatty phase was warmed to 80°C, sunscreen was added, then the mixture was added to water, which contained the hydrosoluble compounds, heated to 80°C. Stirring was maintained for 15-20 minutes. After slow cooling, perfume was added.
**Sun-formulation n. 1:** the sunscreen is the compound A of formula I wherein
   R=(CH₃)₃C-CH₂-C(CH₃)₂-
   R₁=R₂=C₄H₉-CH(C₂H₅)-CH₂; X=O
**Sun-formulation n. 2:** the sunscreen is 3-(4'-methylbenzylidene)camphor (Eusolex 6300) ^{(R)}
**Sun-formulation n. 3:** the sunscreen is a mixture of 3.5 g of compound A and 1.5 g of 3-(4'-methylbenzylidene)camphor.

For each formulation sun protecting factor was determined according to the method described by B. Diffey and J. Robson in J. Soc. Cosmet. Chem. 40, 127-133 (1989).

The Table shows the results:

**TABLE**

| **Sun-formulation** | **SPF** |
|---|---|
| 1 | 6.2 |
| 2 | 4.1 |
| 3 | 7.6 |

### Example 2 - Lotion

| | |
|---|---|
| Compound A | 2.5 g |
| 3-(4'-methylbenzylidene)camphor | 7.5 g |
| Octyl octanoate | 54.0 g |
| Triglycerides C₈-C₁₀ | 32.0 g |
| Dioctylcyclohexane | 10.0 g |
| Perfume | 0.1 g |

The mixture of the solvents was warmed to 60°C, under stirring, the two sunscreens were added, stirring was continued for 10-15 minutes, after cooling perfume was added.

### Example 3 - Oil/Water sun-cream

| | |
|---|---|
| C₁₂-C₁₅ alkyl benzoate | 5.0 g |
| Diisopropyl adipate | 5.0 g |
| Karitè Butter | 2.0 g |
| Bisabalol-A | 0.5 g |
| Compound B | 3.0 g |
| 4-(2'-oxobornylidene-3-methyl)benzensulphonic acid | 3.5 g |
| Stabylen 30 ^{(R)} (Thickening agent 3V-SIGMA) | 0.3 g |
| Synthalen K ^{(R)} (Thickening agent 3V-SIGMA) | 0.3 g |
| Abiol ^{(R)} (Preservative of the 3V-SIGMA) | 0.3 g |
| Methylparaben | 0.2 g |
| Propylparaben | 0.1 g |
| Glycerin | 5.0 g |
| Aminomethylpropanol | 0.5 g |
| Water | q.s. to 100.0 |
| Perfume | |

Compound B is the sunscreen of formula I wherein
R=CH₃-; R₁=R₂=C₄H₉-CH(C₂H₅)-CH₂-; X=O

Fatty phase was warmed to 70°C, the sunscreens were added and stirring was continued for 10-15 minutes. Stabylen 30 and Synthalen K were dispersed in water and the resulting aqueous dispersion was heated to 70°C, the fatty phase was added under strong stirring. Neutralization with aminomethylpropanol followed, cooling to 35°C and preservatives, glycerin and perfume were added.

### Example 4 - Oil/water day-cream

| | |
|---|---|
| Triglyceryl methylglucose distearate | 4.0 g |
| Glyceryl stearate | 1.0 g |
| C₁₂-C₁₅ alkyl benzoate | 7.4 g |
| Avocado oil | 5.0 g |
| Diisopropyl adipate | 5.0 g |
| Compound C | 1.5 g |
| 3-(4'-methylbenzylidene)camphor | 3.5 g |
| Synthalen K (Thickening agent by 3V-SIGMA) | 0.2 g |
| Abiol (Preservative by 3V-SIGMA) | 0.3 g |
| Methylparaben | 0.2 g |
| Propylparaben | 0.1 g |
| Aminomethyl propanol | 0.15 g |
| Glycerin | 3.0 g |
| Distilled water | q.s. to 100.0 |
| Perfume | |

Compound C is the sunscreen of formula I wherein
R=(CH₃)₃C-; R₁=R₂=C₄H₉-CH(C₂H₅)-CH₂-; X=O
execution was as in Example 3.

### Example 5 - Water/oil sun-milk

| | |
|---|---|
| Hydrogenated castor oil ethoxylated with 7 moles of ethylene oxide | 7.5 g |
| Alcohols of lanoline in mineral oil | 2.5 g |
| Octyl octanoate | 7.5 g |
| Dioctylcyclohexane | 5.0 g |
| Cetylstearyl octanoate | 5.0 g |
| Compound B | 3.0 g |
| 3-(4'-methylbenzylidene)camphor | 5.0 g |
| Abiol (Preservative by 3V-SIGMA) | 0.3 g |
| Glycerin | 5.0 g |
| Water | q.s. to 100.0 |
| Perfume | |

The fatty phase was warmed to 70°C and sunscreens were added. Water, pre-heated to 70°C, was added to the fatty phase, under strong stirring. After cooling, preservative, glycerin and perfume were added.

### Example 6 - Lipstick

The base mixture consisting was first prepared:

| | |
|---|---|
| Beeswax | 13.0 g |
| Carnauba wax | 7.4 g |
| Lanoline | 5.0 g |
| Isopropyl myristate | 8.0 g |
| Mineral oil | 3.0 g |
| Castor oil | 63.5 g |

85 g of this mixture were heated to melt, to the molten mass 7 g of compound A and 7 g of 3-(4'-methylbenzylidene)camphor were added, as well as perfume and dyes, then the mixture was diluted to 1000 g with castor oil and cooled down to room temperature.

## Claims

1. Cosmetic compositions for the protection of human skin from sun radiation, comprising in admixture with conventional vehicles and excipients a combination of at least a compound of formula I 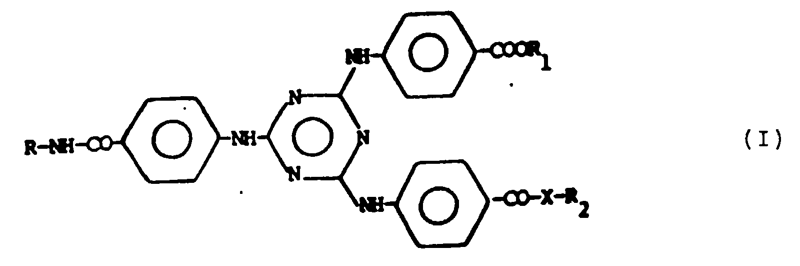 wherein
R is C₁-C₈ straight or branched alkyl, C₅-C₁₂ cycloalkyl, optionally substituted with one or more C₁-C₄ alkyl groups;
X is oxygen or the NH- group;
R₁ has the same meanings of R or is hydrogen, an alkali metal, an ammonium group or a group of formula III 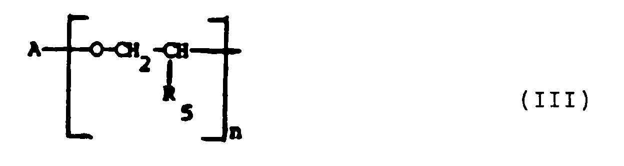 wherein
A is C₁-C₈ straight or branched alkyl, C₅-C₈ cycloalkyl, aryl, optionally substituted with one or more C₁-C₄ alkyls, R₅ is hydrogen or methyl and n can be an integer from 1 to 10;
R₂ has the same meanings of R when X is the NH group or has the same meaning of R₁ when X is oxygen;
with at least a compound of formula II 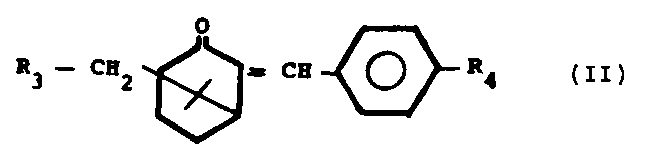 wherein
R₃ is hydrogen, or the SO₃M group, wherein M is an alkali metal or an amine group, R₄ is methyl, a SO₃M group, or a group of formula IV or V

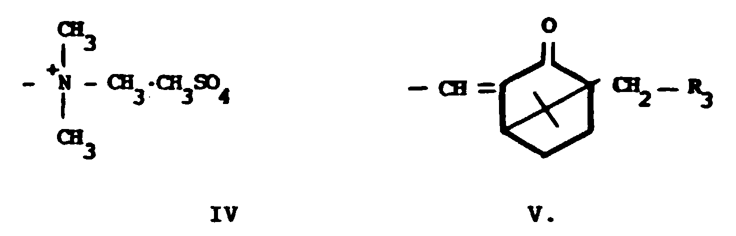

2. Compositions according to claim 1, wherein in the compound of formula I X is oxygen.

3. Compositions according to claim 1, wherein in the compounds of formula I X is oxygen and R₁ and R₂ are C₁-C₈ straight or branched alkyl or C₆-C₁₂ cycloalkyl, optionally substituted with one or more C₁-C₄ alkyls.

4. Compositions according to claims 1-3 containing also zinc oxide.

5. Compositions according to claims 1-3 containing also titanium oxide.

6. Compositions according to claims 1-5 containing also other sunscreens selected from the group consisting of 2-ethylhexyl-2-cyano-3,3-diphenylacrylate; 2-ethylhexyl-4-methoxy-cinnamate; menthylsalicylate; 2,4,6-trianilino-(p-carbo-2-ethylhexyloxy)-1,3,5-triazine; 2-phenylbenzimidazol-5-sulfonic acid; 4-methoxy-4-terbutyldibenzoylmethane; 4-isopropyldibenzoylmethane; 2-hydroxy-4-methoxybenzophenone.

7. Compositions according to anyone of claims 1-6, containing as adjuvants one or more components selected from the group consisting of thickening agents, emollients, hydrating agents, preservatives, perfume.

8. Use of the compositions according to anyone of claims 1-7 for the cosmetic treatment of human skin or hair when exposed to sun radiation.

9. Process for the protection of cosmetic compositions from UV radiations characterized in that from 0.5 to 6% by weight with respect to the composition of a compound of formula I and from 1 to 10% by weight with respect to the composition of a compound of formula II are added to the cosmetic substrate.
